# EUROPEAN PATENT SPECIFICATION

(11) **EP 2 895 195 B1**
(45) Date of publication and mention of the grant of the patent: **08.11.2017**
(21) Application number: 13762107.4
(22) Date of filing: 11.09.2013
(51) Int. Cl.: A61K 47/30, A61K 9/20, C07C 231/00

(54) **STABILISED AMORPHOUS FORM OF AGOMELATINE, A PROCESS FOR ITS PREPARATION AND PHARMACEUTICAL COMPOSITIONS CONTAINING IT**
STABILISIERTE AMORPHE FORM VON AGOMELATIN, VERFAHREN ZU IHRER HERSTELLUNG UND DIESE ENTHALTENDE PHARMAZEUTISCHE ZUSAMMENSETZUNGEN
FORME AMORPHE STABILISÉE D'AGOMÉLATINE, PROCÉDÉ POUR SA PRÉPARATION ET COMPOSITIONS PHARMACEUTIQUES LA CONTENANT

(30) Priority: 11.09.2012 WO PCT/CN2012/081250; 26.09.2012 FR 1259064
(43) Date of publication of application: 22.07.2015
(73) Proprietor: Les Laboratoires Servier, 92284 Suresnes Cedex (FR); Shanghai Institute Of Pharmaceutical Industry, Shanghai 200040 (CN)
(72) Inventor: LAFARGUE, David, 45650 Saint-Jean le Blanc (FR); LYNCH, Michael, 45140 Saint Jean de la Ruelle (FR); POIRIER, Cécile, 45000 Orleans (FR); LETELLIER, Philippe, 45000 Orléans (FR); PEAN, Jean-Manuel, 45000 Orleans (FR); LUO, Ying, Jiangxi 300006 (CN); SHAN, Hanbin, Jiangxi Province Jiangxi 330800 (CN); SHEN, Yuhui, Shanghai 200439 (CN)
(74) Representative: Giudicelli, Cathy
(86) International application number: PCT/EP2013/068792
(87) International publication number: WO 2014/041015

(56) References cited:
- WO-A1-2008/137461
- WO-A1-2012/093402
- CN-A- 101 836 966
- CN-A- 102 716 493

## Description

The present invention relates to the stabilised amorphous form of agomelatine, or *N*-[2-(7-methoxy-1-naphthyl)ethyl]acetamide of formula (I): 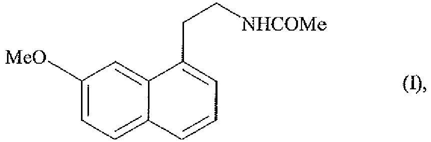 to a process for its preparation and also to pharmaceutical compositions containing it.

Agomelatine, or *N*-[2-(7-methoxy-1-naphthyl)ethyl]acetamide, has valuable pharmacological properties.

In fact, it has the double characteristic of being, on the one hand, an agonist of receptors of the melatoninergic system and, on the other hand, an antagonist of the 5-HT_{2C} receptor. These properties provide it with activity in the central nervous system and, more especially, in the treatment of major depression, seasonal affective disorder, sleep disorders, cardiovascular pathologies, pathologies of the digestive system, insomnia and fatigue due to jet-lag, appetite disorders and obesity.

Agomelatine, its crystalline forms, its complexes, its co-crystals, its addition salts with a pharmaceutically acceptable acid or base, its preparation and its use in therapeutics have been described in the patent applications EP0447285, WO2005/077887, WO2007/015003, WO2007/015002, WO2007/015004, WO2010/097052, WO2010/102554, CN101955440, WO2011/050742, CN102050756, WO2011/006387, WO2011/075943, CN101774937, WO2011/006387, CN101870662 and CN102030673.

Agomelatine is moreover an active ingredient which has the disadvantage of having a very low bioavailability and, as a consequence, a large inter-individual variability. This low bioavailability is in part related to the solubility of agomelatine in water, which is less than 0.15 mg/ml at 25°C.

In view of the pharmaceutical value of this compound and its poor bioavailability, the amorphous form seems to be a possible strategy, as amorphous forms of solid compounds are known to have better dissolution properties than their corresponding crystalline forms.

The Applicant has now discovered that agomelatine has an amorphous state whose glass transition temperature has been measured at -6°C by scanning calorimetric analysis, or DSC ("Differential Scanning Calorimetry"). Above that temperature, the active ingredient undergoes a transition towards a crystalline form. Preserving agomelatine in the amorphous state at ambient temperature is therefore not possible.
Accordingly, the technical problem posed is to make available the amorphous form of agomelatine in a formulation which is compatible with its use in the pharmaceutical industry, especially in terms of stability and storage under customary conditions.

A solution known to the person skilled in the art consists of forming a dispersion of the active ingredient in a solid matrix which surrounds the molecules and prevents them from forming a crystal lattice. The more dilute the active ingredient within the matrix, the greater the amorphous formation. A consequence is that the size of tablets containing the amorphous active ingredient increases substantially with the matrix, which constitutes a major drawback for the patient who has to swallow them. Accordingly, another challenge lies in minimising the amount of matrix used, to the benefit of the active ingredient, whilst still preventing the crystal lattice from forming.
Tests have been carried out with agomelatine in cyclodextrins or trehalose, which are customarily used to form complexes of the "host-guest" type, but it has never been possible to obtain, in a manner that is stable over time, the type of complexes sought.

The Applicant has now discovered that it is possible to obtain a stable formulation of agomelatine in amorphous form in reproducible and industrially feasible manner. This new stabilised form allows to envisage its use in the pharmaceutical industry. It moreover allows high active ingredient contents, resulting in a final size of formulation which is entirely compatible with its being taken by the patient.
The present invention thus relates to the stabilised amorphous form of agomelatine. By "stabilised" it is understood that the amorphous form of agomelatine is preserved when it is subjected to denaturing storage conditions of temperature and humidity for at least one week. The denaturing conditions of temperature and humidity according to the invention will be on average, for example 40°C / 75% RH (relative humidity), 30°C / 65% RH, 50°C, 70°C...

More specifically, the present invention consists of a solid dispersion of agomelatine within an organic polymer, it being understood that the agomelatine represents at least 30 % by weight of the dispersion. It is possible, depending on the organic polymer chosen, to increase this percentage by weight of agomelatine. The solid dispersions wherein the percentage by weight of agomelatine is from 30 to 50%, and more preferably from 30 to 40% are preferred. Surprisingly, even at those high active ingredient contents, the amorphous form of agomelatine is preserved - in a manner that is stable over time. Moreover, besides the improvement in dissolution rate which could be expected customarily with amorphisation of the active ingredient, the solubility of the agomelatine obtained is also greatly increased well beyond the solubility measured for crystalline agomelatine, with its being at least tripled and in some cases multiplied by a factor of 8. This result is entirely surprising because although numerous publications in the field describe an improvement in the biopharmaceutical performance of active ingredients (solubility, dissolution rate) by designing stable solid dispersions this is for loadings of less than 20 % of active ingredient (Lin et al., International Journal of Pharmaceutics, 1996, 127, 261-272).
Surprisingly, the increase in solubility of the active ingredient is maintained for at least 4 hours.

The polymer used in the present invention relates more especially to methacrylic acid compounds or vinyl or cellulosic polymers.

More especially, the polymers used according to the invention relate to polymethacrylates or copolymers of methacrylic acid which correspond to a completely polymerised copolymer of methacrylic acid and acrylic or methacrylic ester. These polymethacrylates are customarily referred to as Eudragit® and may be in the form of a powder or granules. Among the different Eudragit® products marketed, those preferably used in the context of the invention are Eudragit® L products, more especially Eudragit® L100 and L100-55, or Eudragit® EPO. These polymers are particularly adapted to all ranges of agomelatine loading (% by weight of agomelatine).

The vinyl polymers according to the invention relate more especially to polyvinyl esters such as, for example, polyvinyl acetate phthalate; homo- or co-polymers polyvinylpyrrolidone based such as Povidones (Povidone® K30, Plasdone® S630), Kollidon VA64 or also Soluplus®. Povidones will be used for loading inferior to 50% by weight of agomelatine.

Among the cellulosic compounds used according to the invention there may be mentioned cellulosic ethers or esters such as HPMCs, and more especially HPMC acetyl succinate.

The present invention relates also to a process for obtaining a stable formulation having a high content of the amorphous form of agomelatine within a polymer. According to the process of the invention, the compound of formula (I) obtained by any process and present in any crystalline form, complexes, co-crystals, or addition salts with a pharmaceutically acceptable acid or base, is mixed with the selected polymer in one or more solvents, allowing complete dissolution of the constituents to be obtained, and then the solvent is evaporated off in its entirety under reduced pressure.
The solvents used according to the invention are those capable of dissolving agomelatine and the selected polymer; preference will be given to polar protic or aprotic solvents such as acetone, alcohols and more especially methanol and ethanol, water, dichloromethane, ethyl acetate or mixtures of those solvents. Dissolution is carried out with stirring at ambient temperature or by heating the mixture until dissolution of the constituents is complete. Evaporating off the solvents is carried out under reduced pressure at ambient temperature or by heating until evaporation of the solvents is complete.

An advantageous embodiment of the process for preparation of a stable formulation having a high content of the amorphous form of agomelatine within a polymer consists of mixing and pre-blending the compound of formula (I) obtained by any process and present in any crystalline form, complexes, co-crystals, or addition salts with a pharmaceutically acceptable acid or base, with the selected polymer and then this mixture is introduced into an extruder whose screw pitch and temperature are chosen as a function of the viscosity of the mixture, to obtain an extrudate which is then cut up into the desired size and then, optionally, ground.
Preferably, rotation of the screw will be carried out between 50 and 200 rpm, more especially between 75 and 150 rpm.
The extrusion temperature selected will be a function of the viscosity of the resulting mixture of constituents and will be between 90°C and 200°C inclusive.

In the processes for obtaining the stabilised amorphous form of agomelatine according to the invention, the compound of formula (I) obtained by any process and present in any crystalline form, complexes, co-crystals, or addition salts with a pharmaceutically acceptable acid or base, may be used.
In the processes according to the invention, the agomelatine loading is greater than or equal to 30 % by weight and more especially varies from 30 to 50 % by weight, preferably from 30 % to 40 %.

The stabilised amorphous form thereby obtained has value in the treatment of disorders of the melatoninergic system and has shown substantial activity on the central nervous system and microcirculation, making it possible to establish its usefulness in the treatment of stress, sleep disorders, anxiety, major depression, seasonal affective disorder, bipolar disorder, generalised anxiety disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders. In another field of activity, it appears that, in treatment, the amorphous form of agomelatine can be used in sexual dysfunctions, that it has ovulation-inhibiting and immunomodulating properties and that it may potentially be used in the treatment of cancers.

The stabilised amorphous form of agomelatine will preferably be used in treating major depression, seasonal affective disorder, bipolar disorder, generalised anxiety disorder, sleep disorders, cardiovascular pathologies, pathologies of the digestive system, insomnia and fatigue due to jet-lag, appetite disorders and obesity.

Obtaining the stabilised amorphous form of agomelatine has the advantage of making possible the preparation of pharmaceutical formulations of consistent and reproducible composition, which have excellent stability over time.

According to the invention it is therefore possible to obtain solid pharmaceutical compositions having high contents of the amorphous form of agomelatine, which are administrable especially by the oral, buccal, sublingual, ocular, rectal, vaginal or parenteral route.

These pharmaceutical compositions can be made of the solid dispersion of agomelatine within the polymer without any other processing operation besides packaging. If desired, said pharmaceutical compositions may, however, undergo processing by grinding or by granulation for filling into capsules or for compression or may undergo coating.

The pharmaceutical compositions of the invention may also optionally comprise pharmacologically acceptable excipients selected, for example, from the group of binders, disintegrating agents, disintegrants, lubricants, diluents, antioxidants, aromatic agents, colourants, preservatives, sweeteners and anti-adherents.

Among the pharmaceutical compositions according to the invention there may be more especially mentioned tablets or dragées, granules, sublingual tablets, capsules, lozenges, suppositories, creams, ointments, dermal gels, injectable preparations, drinkable suspensions and chewing gums.

Preferably, the pharmaceutical compositions according to the invention contain at least 25% by weight of agomelatine with respect to the total weight of the formulation.

The useful dosage can be varied according to the nature and severity of the disorder, the administration route and the age and weight of the patient. The dosage varies from 0.1 mg to 1 g per day of agomelatine in one or more administrations.

### Description of the Figures

Fig.1: X-ray diffraction diagrams of Example 10 recorded under various stability conditions (1 week at 40°C 75% RH, 1 week at 40°C / 75% RH then 1 week at 50°C, 1 week at 70°C)
Fig.2: X-ray diffraction diagrams of Example 11 recorded under various stability conditions (1 week at 40°C 75% RH, 1 week at 40°C / 75% RH then 1 week at 50°C, 1 week at 70°C)
Fig.3: X-ray diffraction diagrams of Example 12 recorded under various stability conditions (1 week at 40°C 75% RH, 1 week at 40°C / 75% RH then 1 week at 50°C, 1 week at 70°C)
Fig.4: X-ray diffraction diagrams of Example 19 recorded under various stability conditions (1 week at 40°C 75% RH, 1 week at 40°C / 75% RH then 1 week at 50°C, 1 week at 70°C)
Fig.5: X-ray diffraction diagrams of Example 28 recorded under various stability conditions (1 week at 40°C 75% RH, 1 week at 40°C / 75% RH then 1 week at 50°C, 1 week at 70°C)
Fig.6: X-ray diffraction diagrams recorded at the end of 3 months under various stability conditions (25°C / 60 % RH in an open or closed flask, 30°C / 65 % RH in an open or closed flask, 50°C in an open flask) of Example 39
Fig.7: X-ray diffraction diagrams recorded at the end of 3 months under various stability conditions (25°C / 60 % RH in an open or closed flask, 30°C / 65 % RH in an open or closed flask, 50°C in an open flask) of Example 40

The Examples hereinbelow illustrate the invention but do not limit it in any way.

### A. General procedure for obtaining the stabilised amorphous form of agomelatine by dissolution-evaporation

Agomelatine and the selected polymer are placed in a 10-ml vial with 3 ml of solvent(s). The mixture is stirred at 40°C for 30 minutes until dissolution is complete and a homogeneous solution has been obtained. The solvent(s) is/are evaporated off under reduced pressure at 40°C for 30 minutes. The residue obtained is finally dried *in vacuo* at ambient temperature (20°C) overnight (12 hours) to yield the stabilised amorphous form of agomelatine. For each test, the stability of the amorphous form was tested under denaturing conditions at 40°C and 75 % RH, at 40°C and 75 % RH followed by 50°C, and also at 70°C.

The various Examples produced and results obtained are set out in the Table below:

| **Example** | Agomelatine % by weight | Polymer | Solvents | Stability under denaturing conditions | | |
|---|---|---|---|---|---|---|
| | | | | 1 week at 40°C, 75%RH | 1 week at 40°C, 75%RH, then 1 week at 50°C | 1 week at 70°C |
| **1** | 30 | Eudragit L100-55 | Acetone | amorphous | amorphous | amorphous |
| **2** | | | EtOH | | | |
| **3** | | | Acetone /EtOH 70/30 | | | |
| **4** | 35 | | Acetone | amorphous | amorphous | amorphous |
| **5** | | | EtOH | | | |
| **6** | | | Acetone /EtOH 70/30 | | | |
| **7** | 40 | | Acetone | amorphous | amorphous | amorphous |
| **8** | | | EtOH | | | |
| **9** | | | Acetone /EtOH 70/30 | | | |
| **10** | 50 | | Acetone | amorphous | amorphous | amorphous |
| **11** | | | EtOH | | | |
| **12** | | | Acetone /EtOH 70/30 | | | |
| **13** | 30 | Kollidon VA 64 | Acetone | amorphous | amorphous | amorphous |
| **14** | | | EtOH | | | |
| **15** | | | Acetone /EtOH 70/30 | | | |
| **16** | 35 | | Acetone | amorphous | amorphous | amorphous |
| **17** | | | EtOH | | | |
| **18** | | | Acetone /EtOH 70/30 | | | |
| **19** | 40 | | Acetone | amorphous | amorphous | amorphous |
| **20** | | | EtOH | | | |
| **21** | | | Acetone /EtOH 70/30 | | | |
| **22** | 30 | Soluplus | Acetone | amorphous | amorphous | amorphous |
| **23** | | | EtOH | | | |
| **24** | | | Acetone /EtOH 70/30 | | | |
| **25** | 35 | | Acetone | amorphous | amorphous | amorphous |
| **26** | | | EtOH | | | |
| **27** | | | Acetone /EtOH 70/30 | | | |
| **28** | 40 | | Acetone | amorphous | amorphous | amorphous |
| **29** | | | EtOH | | | |
| **30** | | | Acetone /EtOH 70/30 | | | |

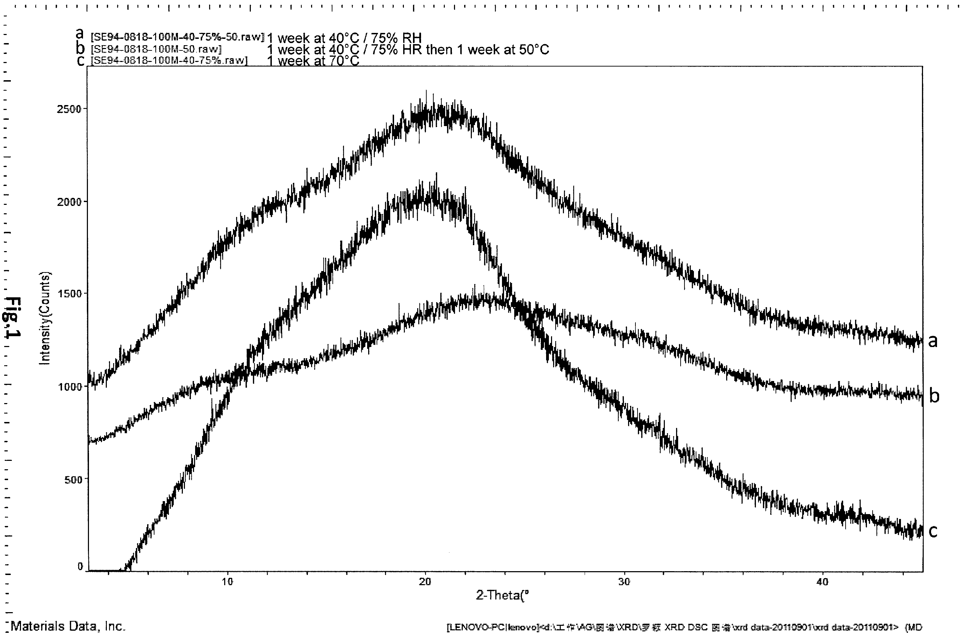

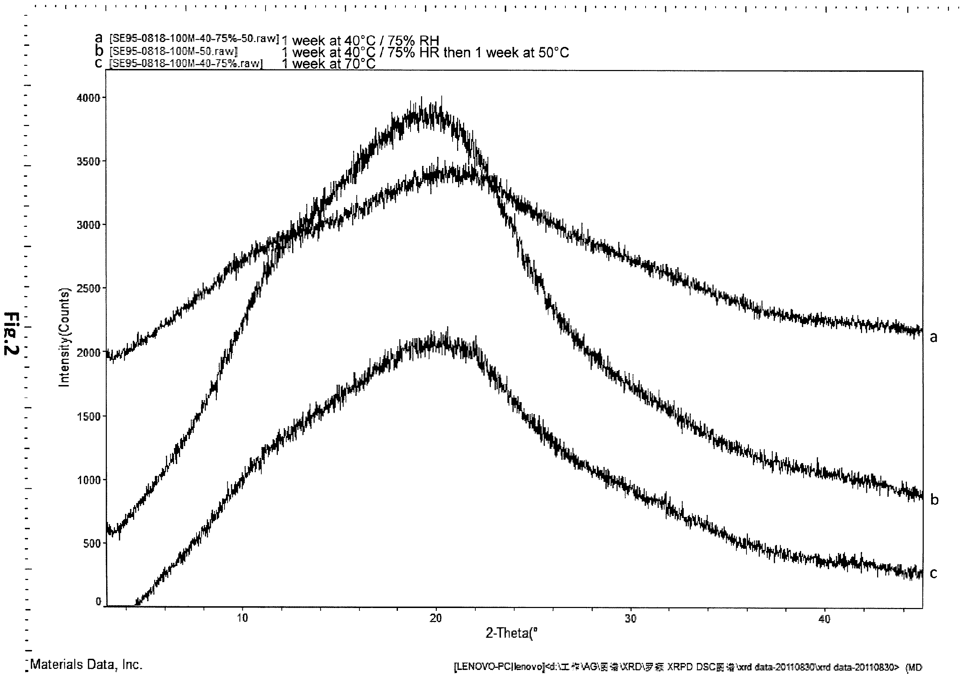

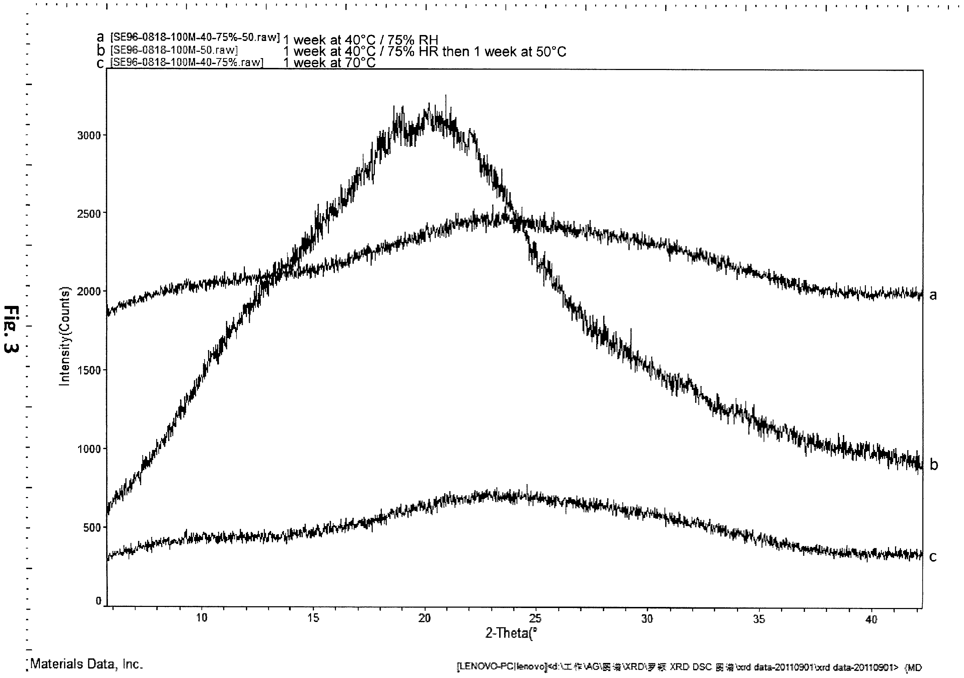

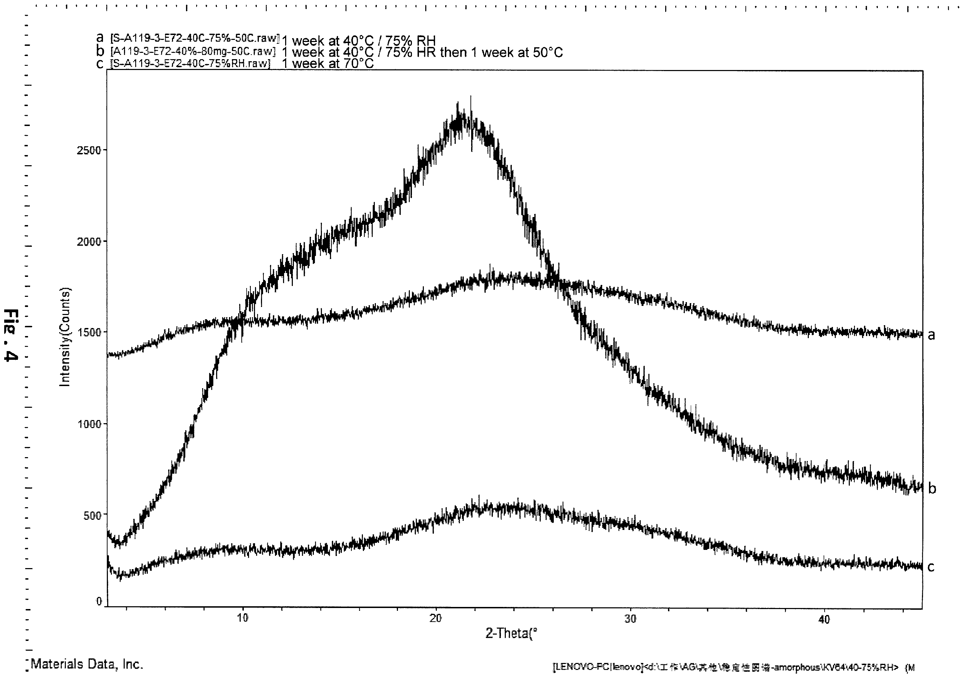

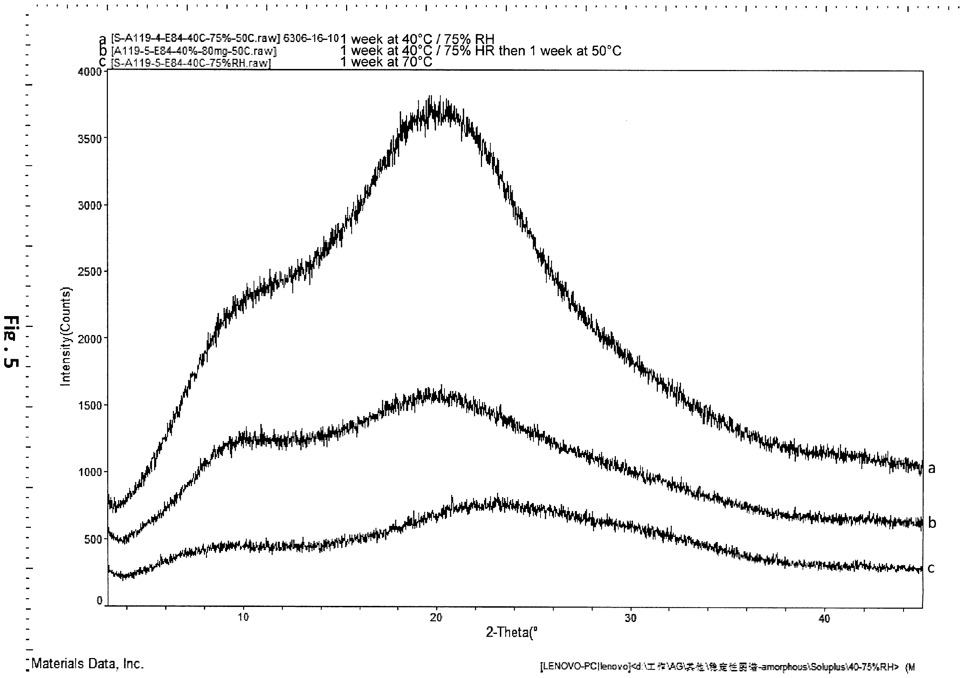

By way of example, the X-ray diffraction diagrams of examples 10, 11, 12, 19 and 28 recorded under various stability conditions (1 week at 40°C 75% RH, 1 week at 40°C / 75% RH then 1 week at 50°C, 1 week at 70°C) are set out in Fig.1, Fig.2, Fig.3, Fig.4 and Fig.5 respectively.

### B. General process for obtaining the stabilised amorphous form of agomelatine by extrusion

Agomelatine and the polymer are pre-blended in a Turbula-type mixer for 10 minutes. The mixture obtained is placed manually in a conical rotor extruder (diameter 5/14mm) of the HAAKE Minilab II Microcompounder type (ThermoFisher). The extrusion speed is 100 rpm.

The stability of the amorphous form obtained was assessed under various temperature and relative humidity conditions: 25°C / 60 % RH in an open or closed flask, 30°C / 65 % RH in an open or closed flask, 50°C in an open flask.

All the Examples given in the Table below have stabilities of more than at least 6 weeks.

| **Example** | Agomelatine % by weight | Polymer | Extrusion temperature |
|---|---|---|---|
| **31** | 30 | Eudragit L100 | 190°C |
| **32** | 40 | | |
| **33** | 50 | | |
| **34** | 30 | Eudragit L100-55 | 150°C |
| **35** | 40 | | |
| **36** | 50 | | |
| **37** | 30 | HPMC acetyl succinate | 135°C |
| **38** | 30 | Plasdone S630 | 110°C |
| **39** | 30 | Polyvinyl acetate phthalate | 150°C |
| **40** | 40 | | |
| **41** | 30 | Povidone K30 | 140°C |

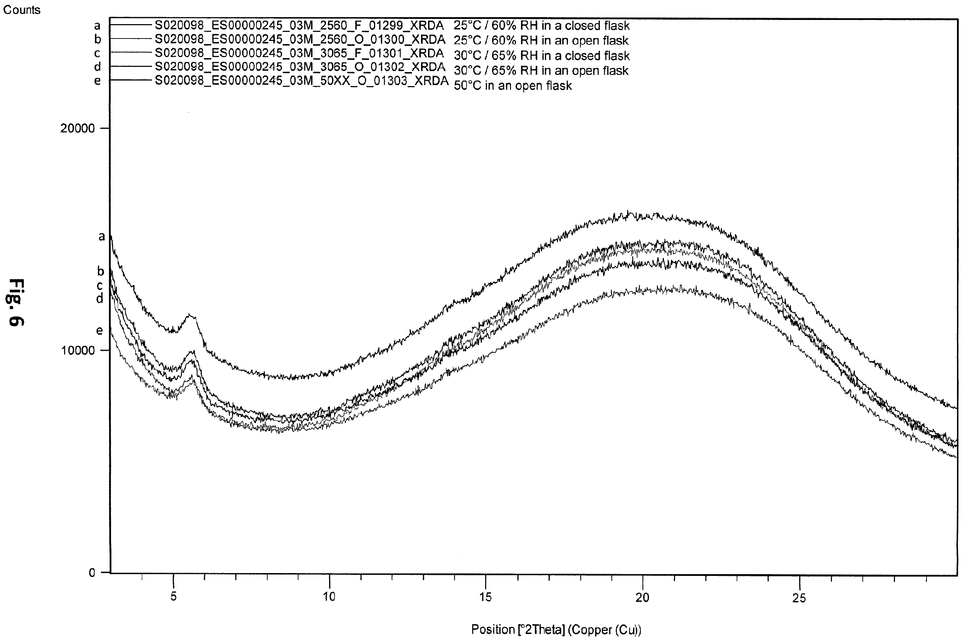

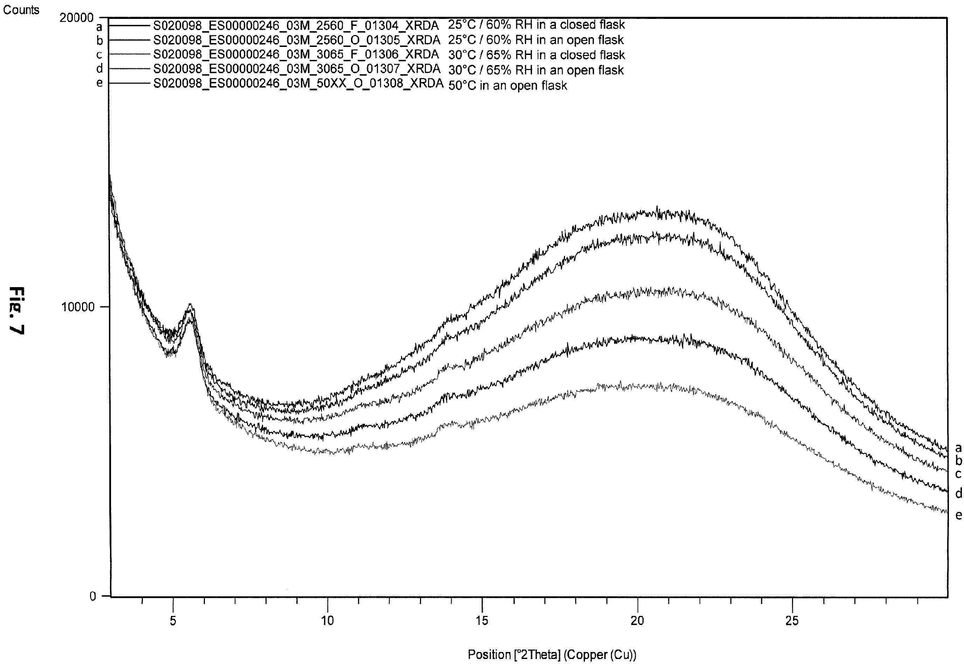

By way of example, the X-ray diffraction diagrams recorded at the end of 3 months under various stability conditions (25°C / 60 % RH in an open or closed flask, 30°C / 65 % RH in an open or closed flask, 50°C in an open flask) for Examples 39 and 40 are set out in Fig.6 and Fig.7 respectively.

### C. Solubility

A solubility study of the formulations obtained was carried out using a Crystal 16® type apparatus in a pH 6.8 buffer solution at 25°C over a period of 4 hours with a stirring speed of 700 rpm. Various concentrations were tested and the presence of insoluble particles was monitored by nephelometric detection. As reference, the solubility of agomelatine under those conditions is 0.14 mg/ml.

The results obtained are set out in the Table below, which sets out i) the maximum solubility observed and the time at which this maximum solubility is observed, ii) the solubility observed at 4 hours.

| **Example** | Max. solubility (mg/ml) observed at t minutes | Solubility (mg/ml) at 4 hours |
|---|---|---|
| **14** | > 0.9 at 5 mins. | > 0.59 |
| **20** | > 0.9 at 6 mins. | > 0.42 |
| **31** | > 1.5 at 5 mins. | > 1.20 |
| **32** | > 1.2 at 45 mins. | > 0.46 |
| **33** | > 1.1 at 10 mins. | > 0.52 |
| **34** | > 0.67 at 5 mins. | > 0.24 |
| **35** | > 0.9 at 5 mins. | > 0.40 |
| **36** | > 0.76 at 10 mins. | > 0.33 |
| **37** | > 0.79 at 5 mins. | > 0.32 |
| **38** | > 0.34 at 5 mins. | > 0.34 |
| **39** | > 0.64 at 5 mins. | > 0.43 |
| **40** | > 0.53 at 5 mins. | > 0.29 |
| **41** | > 0.73 at 5 mins. | > 0.37 |

The results obtained show a substantial increase in the maximum solubility. The important point is that this solubility increase continues over time: at 4 hours, the results show that it is at least multiplied by a factor ranging from 1.7 to 8.5, which gives time for the active ingredient to be absorbed before it is reprecipitated.

### D. Pharmaceutical compositions

### Example 42

Formula for the preparation of 1000 capsules each containing 25 mg of agomelatine:

| | |
|---|---|
| Agomelatine | 25 g |
| Eudragit L100-55 | 25 g |

The extrudate is prepared in accordance with Example 36 and is then cut into a mini-matrix and introduced into a size 1 capsule.

### Example 43

Formula for the preparation of 1000 capsules each containing 25 mg of agomelatine:

| | |
|---|---|
| Agomelatine | 25 g |
| Plasdone S630 | 58 g |

The extrudate is prepared in accordance with Example 38 and is then cut into a mini-matrix and introduced into a size 1 capsule.

### Exemple 44

Formula for the préparation of 1000 tablets each containing 25 mg of agomélatine :

| | |
|---|---|
| Extrudat Exemple 36 | 50 g |
| Maize starch | 10 g |
| Lactose | 20 g |
| Magnésium stearate | 0,5 g |
| Silica | 0,25 g |
| Hydroxypropylcellulose | 2,25 g |

## Claims

1. Stabilised amorphous form of agomelatine of formula (I): 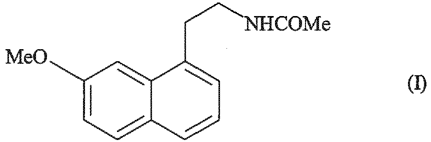 **characterised in that** agomelatine is dispersed within a matrix as follows:
- 30%, 40% or 50% by weight of agomelatine in Eudragit L100,
- 30%, 35%, 40% or 50% by weight of agomelatine in Eudragit L100-55,
- 30%, 35% or 40% by weight of agomelatine in Kollidon VA64,
- 30%, 35% or 40% by weight of agomelatine in Soluplus,
- 30% or 40% by weight of agomelatine in Polyvinyl Acetate Phthalate,
- 30% by weight of agomelatine in Plasdone S630, and
- 30% by weight of agomelatine in HPMC acetyl succinate.

2. Process for obtaining the stabilised amorphous form of agomelatine according to claim 1, **characterised in that** the compound of formula (I) obtained by any process and present in any crystalline form, complexes, co-crystals, or addition salts with a pharmaceutically acceptable acid or base, is mixed with the selected polymer in one or more solvents, allowing complete dissolution of the constituents to be obtained, and then the solvent is evaporated off in its entirety under reduced pressure.

3. Process for obtaining the stabilised amorphous form of agomelatine according to claim 1, **characterised in that** the compound of formula (I) obtained by any process and present in any crystalline form, complexes, co-crystals, or addition salts with a pharmaceutically acceptable acid or base, is mixed and pre-blended with the selected polymer and then is introduced into an extruder whose screw pitch and temperature are chosen as a function of the viscosity of the mixture, to obtain an extrudate which is then cut up into the desired size and then, optionally, ground.

4. Pharmaceutical compositions comprising as active ingredient the stabilised amorphous form of agomelatine according to claim 1, on its own or in combination with one or more inert, non-toxic and pharmaceutically acceptable carriers.

5. Pharmaceutical compositions according to claim 4 , **characterized in that** the percentage of agomelatine by weight with respect to the total weight of the formulation is greater than or equal to 25%.

6. Stabilised morphous form of agomelatine according to claim 1 for use in the treatment of disorders of the melatoninergic system.

7. Stabilised amorphous form of agomelatine according to claim 1 for use in the treatment of sleep disorders, stress, anxiety, seasonal affective disorder or major depression, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, cerebral circulation disorders and also in sexual dysfunctions, and as ovulation-inhibitors and immunomodulators and in the treatment of cancers.

## Patentansprüche

1. Stabilisierte amorphe Form von Agomelatin der Formel (I): 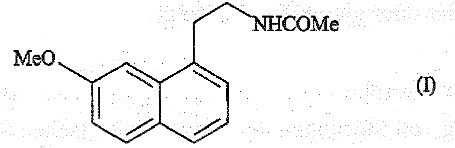 **dadurch gekennzeichnet, dass** Agomelatin wie folgt in einer Matrix dispergiert ist:
- 30 %, 40 % oder 50 Gew.-% Agomelatin in Eudragit L100,
- 30 %, 35 %, 40 % oder 50 Gew.-% Agomelatin in Eudragit L100-55,
- 30 %, 35 % oder 40 Gew.-% Agomelatin in Kollidon VA64,
- 30 %, 35 % oder 40 Gew.-% Agomelatin in Soluplus,
- 30 % oder 40 Gew.-% Agomelatin in Polyvinylacetatphthalat,
- 30 Gew.-% Agomelatin in Plasdone S630 und
- 30 Gew.-% Agomelatin in HPMC-Acetylsuccinat.

2. Verfahren zur Herstellung der stabilisierten amorphen Form von Agomelatin nach Anspruch 1, **dadurch gekennzeichnet, dass** die nach irgendeinem Verfahren erhaltene und in irgendeiner Form von Kristallen, Komplexen, Co-Kristallen oder Additionssalzen mit einer pharmazeutisch annehmbaren Säure oder Base vorliegende Verbindung der Formel (I) mit dem ausgewählten Polymer in einem oder mehreren Lösungsmitteln vermischt wird, die vollständige Auflösung der Bestandteile erreicht wird und das Lösungsmittel dann unter vermindertem Druck vollständig verdampft wird.

3. Verfahren zur Herstellung der stabilisierten amorphen Form von Agomelatin nach Anspruch 1, **dadurch gekennzeichnet, dass** die mit Hilfe irgendeines Verfahrens erhaltene und in irgendeiner Form von Kristallen, Komplexen, Co-Kristallen oder Additionssalzen mit einer pharmazeutisch annehmbaren Säure oder Base vorliegende Verbindung der Formel (I) mit dem ausgewählten Polymer vermischt und vorvermengt wird und dann in einen Extruder eingeführt wird, dessen Schneckensteigung und Temperatur derart in Abhängigkeit von der Viskosität der Mischung ausgewählt werden, dass ein Extrudat erhalten wird, welches dann zu der gewünschten Größe aufgeteilt und dann gegebenenfalls gemahlen wird.

4. Pharmazeutische Zubereitungen umfassend als Wirkstoff die stabilisierte amorphe Form von Agomelatin nach Anspruch 1 allein oder in Kombination mit einem oder mehreren inerten, nichttoxischen und pharmazeutisch annehmbaren Hilfsstoffen.

5. Pharmazeutische Zubereitungen nach Anspruch 4, **dadurch gekennzeichnet, dass** der Gewichtsprozentsatz von Agomelatin bezogen auf das Gesamtgewicht der Formulierung größer oder gleich 25 % beträgt.

6. Stabilisierte amorphe Form von Agomelatin nach Anspruch 1 zur Verwendung bei der Behandlung von Störungen des melatoninergischen Systems.

7. Stabilisierte amorphe Form von Agomelatin nach Anspruch 1 zur Verwendung bei der Behandlung von Schlafstörungen, Stress, Angst, saisonal bedingten Störungen oder Major-Depression, kardiovaskulären Erkrankungen, Erkrankungen des Verdauungssystems, Schlaflosigkeit und Müdigkeit als Folge von Jetlag, Schizophrenie, Panikattacken, Melancholie, Appetitstörungen, Fettsucht, Schlaflosigkeit, Schmerzen, psychotischen Störungen, Epilepsie, Diabetes, der Parkinsonschen Krankheit, seniler Demenz, verschiedenen Störungen, die mit dem normalen und pathologischen Altern verknüpft sind, Migräne, Gedächtnisverlust, der Alzheimerschen Krankheit, Zirkulationsstörungen des Gehirns und sexuellen Dysfunktionen sowie als Ovulationsinhibitoren und Immunomodulatoren und zur Behandlung von Krebserkrankungen.

## Revendications

1. Forme amorphe stabilisée d'agomélatine de formule (I) : 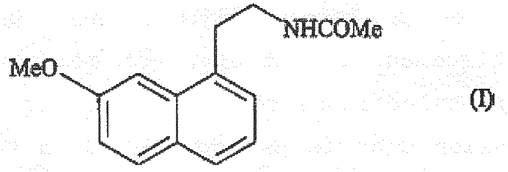 **caractérisée en ce que** l'agomélatine est dispersée au sein d'une matrice comme suit :
- 30 %, 40 % ou 50 % en poids d'agomélatine dans l'Eudragit L100,
- 30 %, 35 %, 40 % ou 50 % en poids d'agomélatine dans l'Eudragit L100-55,
- 30 %, 35 % ou 40 % en poids d'agomélatine dans le Kollidon VA64,
- 30 %, 35 % ou 40 % en poids d'agomélatine dans le Soluplus,
- 30 % ou 40 % en poids d'agomélatine dans l'acétate phtalate de polyvinyl,
- 30 % en poids d'agomélatine dans la Plasdone S630, et
- 30 % en poids d'agomélatine dans l'acétylsuccinate de HPMC.

2. Procédé d'obtention de la forme amorphe stabilisée d'agomélatine selon la revendication 1, **caractérisé en ce que** le composé de formule (I) obtenu par n'importe quel procédé et présent sous n'importe quelle forme cristalline, de complexes, de co-cristaux, ou de sels d'addition avec un acide ou une base pharmaceutiquement acceptable, est mélangé avec le polymère sélectionné dans un ou plusieurs solvants, pour permettre la dissolution complète des constituants, et ensuite le solvant est éliminé par évaporation dans sa totalité sous pression réduite.

3. Procédé d'obtention de la forme amorphe stabilisée d'agomélatine selon la revendication 1, **caractérisé en ce que** le composé de formule (I) obtenu par n'importe quel procédé et présent sous n'importe quelle forme cristalline, de complexes, de co-cristaux, ou de sels d'addition avec un acide ou une base pharmaceutiquement acceptable, est mélangé et pré-malaxé avec le polymère sélectionné et ensuite est introduit dans une machine d'extrusion dont le pas de vis et la température sont choisis en fonction de la viscosité du mélange, pour obtenir un extrudat qui est ensuite coupé à la taille souhaitée et ensuite, facultativement, broyé.

4. Compositions pharmaceutiques comprenant comme principe actif la forme amorphe stabilisée d'agomélatine selon la revendication 1, seule ou en combinaison avec un ou plusieurs supports inertes, non toxiques et pharmaceutiquement acceptables.

5. Compositions pharmaceutiques selon la revendication 4, **caractérisée en ce que** le pourcentage d'agomélatine en poids par rapport au poids total de la formulation est supérieur ou égal à 25 %.

6. Forme amorphe stabilisée d'agomélatine selon la revendication 1 pour son utilisation dans le traitement des troubles du système mélatoninergique.

7. Forme amorphe stabilisée d'agomélatine selon la revendication 1 pour son utilisation dans le traitement des troubles du sommeil, du stress, de l'anxiété, des troubles affectifs saisonniers ou de la dépression sévère, des maladies cardiovasculaires, des maladies du système digestif, des insomnies et de la fatigue provoquées par le décalage horaire, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, de divers troubles associés à un vieillissement normal ou pathologique, de la migraine, des pertes de mémoire, de la maladie d'Alzheimer, des troubles de la circulation cérébrale et également des dysfonctionnements sexuels, et comme inhibiteurs de l'ovulation et immunomodulateurs et dans le traitement des cancers.
